# EUROPEAN PATENT APPLICATION

(11) **EP 4 462 445 A1**
(43) Date of publication of application: **13.11.2024**
(21) Application number: 23200907.6
(22) Date of filing: 29.09.2023
(51) Int. Cl.: G16H 50/20, G16H 50/70, G16H 30/20

(54) **METHOD FOR PROVIDING MEDICAL IMAGING DECISION SUPPORT DATA**

(71) Applicant: Siemens Healthineers AG, 91301 Forchheim (DE)
(72) Inventor: Katzmann, Alexander, 90765 Fürth (DE); Taubmann, Oliver, 91365 Weilersbach (DE); Sühling, Michael, 91052 Erlangen (DE); Kaergel, Rainer, 96135 Stegaurach (DE)
(74) Representative: Siemens Healthineers Patent Attorneys

(57) **Abstract**

In one aspect the invention relates to a computer-implemented method for providing medical imaging decision support data, the method comprising:
- receiving natural language data, the natural language data comprising patient-specific clinical information,
- generating structured information by applying a large language model onto the natural language data, the structured information comprising the patient-specific clinical information in a structured format,
- calculating the medical imaging decision support data based on the structured information, and
- providing the medical imaging decision support data.

## Description

In one aspect the invention relates to a computer-implemented method for providing medical imaging decision support data. In other aspects, the invention relates to a data processing system, a medical imaging device, a computer program product and a computer-readable storage medium.

In clinical applications, making quick and accurate decisions is of utmost importance. As resulting from practical requirements, e.g., time schedules and needs for medical equipment, a typical clinical assessment is not necessarily conducted in a patient-, but often in a specialty-centered fashion. That is, patients are delegated to different specialties as required by an indication as it is derived by the anamnesis up to this point.

As a result, patient-specific clinical information must be steadily passed on to the next point of treatment, either among different diagnostic institutions or between clinical specialties within the same institution. Although there is a vast variety of unifying medical vocabulary, treatment and disease codes, protocols, etc., directly treatment-relevant information may be lost in medical assessments, with many patients receiving a delayed or disrupted treatment and/or being thereby exposed to an additional risk of harm.

Therefore, utilizing pre-defined and well-structured templates that give a blueprint for the required data is key in order to ensure the availability of all required clinical information. While dedicated techniques exist for some specific diseases, like TNM staging and RECIST protocols for cancer diseases, RADS variants (e.g., CAD-RADS or Bone-RADS), and similar - the use of unified and structured reporting of information in clinical environments is typically rare. And even when an appropriate scheme exists as in the aforementioned examples, the way this information is actually represented may still vary significantly in practice.

A main reason for this is the lack of unified software solutions for passing on clinical information and, thus, the reliance on various means of communication. Clinical IT landscapes tend to be highly diverse, and despite unified communication protocols, such as FHIR and DICOM, solutions are often vendor-specific or contain vendor-specific extensions and therefore cannot be universally used within clinics. Moreover, additional restrictions may apply due to data security and data privacy laws.

In a typical scanner workflow, a radiologist decides on the imaging exam to be performed based on a free-text clinical indication. After reading the scan, the radiologist documents findings in an unstructured report that later on serves as a basis for disease diagnosis. Relevant steps, such as the choice of the scanning protocol, reading workflow, and the report generation until now can only partly be supported by software, as they are typically unable to handle natural language.

As a result, clinical information is often communicated in an unstructured way, most notably in unstructured clinical assessment reports, or short unstructured reports. Despite clinicians being trained to use highly descriptive, concise, and compact language, these unstructured reports do neither inherently (i.e., by virtue of the technical procedure used to create them) require the clinician to include all relevant information nor do they necessarily serve as a precise source of information about what needs to be assessed in the further process. Thus, the completeness of information in unstructured reports is mainly a result of clinical experience and adherence to guidelines or regulations, and further prone to influences such as disease salience, practitioner fatigue, and time limitations.

Notably, unstructured text-based information not only plays a vital role between clinical institutions and departments but also plays an important role during the scan and post-processing workflow itself. Every decision about the choice of the scan protocol, the chosen image reconstructions, and the automatically applied post-processing algorithms, to name only a few examples, are directly and immediately based on the clinical indication, i.e., the reason for exam, as well as potentially other clinically relevant information about the patient. Notably, the reason for exam might itself be influenced by previous patient exams. Therefore, relevant sources of information for the scan and postprocessing workflow also include prior diagnostic reports. Typically, most of these data are unstructured and must be transformed, i.e., translated, mapped, and protocoled, into a suitable imaging and postprocessing procedure. This transformation may be conducted manually by radiologists and technologists. As with the availability of cutting-edge technologies, such as photon counting CT, the number of available choices is expected to be steadily increasing, automated support of these tasks is of highest clinical importance.

EP 3 451 211 A1 discloses a concept for controlling a medical imaging system based on indication information using an indication ontology.

There have been efforts to create automated solutions to extract key information from unstructured reports for workflow automation. Such methods have typically been built upon keyword search using lexicographical ontologies, such as RadLex. Due to their nature, these methods often suffer from incorrect parsing of information, as they are unable to fully consider the context. As these models do not come with an actual understanding of the environment but rather process a report in a terminology of symbolic representations, they especially struggle with negations and inversions, abbreviations, extra information, and other content that breaks linear language structure. Moreover, these methods must be designed for a specific clinical task, i.e., they can only be applied to a single or a small number of diseases, as the underlying ontology must be augmented by a large variety of additional rules to handle the previously mentioned issues.

Recent measures like the introduction of DICOM TID 1500 for providing a vendor-neutral structure of encoding diagnostic findings exist but are not widely adopted yet. Moreover, they lack a default way of filling in the data and are obtrusive in the everyday clinical workflow. In clinical practice, coding, that is mapping to structured pre-defined medical codes, of findings and indications appears to be not yet widely adopted except for billing. Specifically for the scan and post-processing workflow itself, many sites still largely rely on unstructured text-based information despite their inherent shortcomings.

The underlying technical problem of the invention is to facilitate a medical imaging decision support that is improved in particular with regard to natural language processing aspects. This problem is solved by the subject matter of the independent claims. The dependent claims are related to further aspects of the invention. Independent of the grammatical term usage, individuals with male, female or other gender identities are included within the term.

In one aspect the invention relates to a computer-implemented method for providing medical imaging decision support data, the method comprising:
- receiving natural language data, the natural language data comprising patient-specific clinical information,
- generating structured information by applying a large language model onto the natural language data, the structured information comprising the patient-specific clinical information in a structured format,
- calculating the medical imaging decision support data based on the structured information, and
- providing the medical imaging decision support data.

In particular, the natural language data may comprise the patient-specific clinical information in an unstructured format. For example, the natural language data may comprise an unstructured report, the unstructured report comprising the patient-specific clinical information. The patient-specific clinical information may comprise, for example, information regarding demographic parameters, in particular gender, weight, height and/or age, physiological parameters, previous findings, previous treatments, a clinical history and/or an anamnesis of a patient. The patient-specific clinical information may comprise, for example, a referral request and/or an indication and/or a reason for a medical imaging examination.

The large language model may be configured to recognize and/or understand natural language and, in particular, individual items such as words in input containing natural language and transfer the items into a text output. The large language model may be trained and/or machine-learned. The large language model may be provided, for example, by holding the large language model available in a suitable storge accessible by a computing unit executing the method and/or by downloading it by said computing unit.

The large language model may be used for transforming unstructured information into the structured format and/or vice versa. Typical large language models have a specific strength in summarizing and presenting information in the way the user prompts the large language model to do. They have an inherent capability of extracting information from text. The large language model may be configured for natural language processing (NLP), in particular for NLP-based extraction of the structured information from the natural language data.

For example, the structured information may comprise a structured report, the structured report comprising the patient-specific clinical information in the structured format. The structured format may be, for example, a predefined and/or computer-readable format. The natural language data and/or the structured information may be text-based.

In the structured format, the patient-specific clinical information can be easily extracted and efficiently communicated in a machine-readable format using predefined, automated communication protocols. Notably, by utilizing a large language model capable of language understanding rather than only parsing, the approach circumvents a variety of major issues arising from the use of merely parsing-based approaches, such as the mentioned inversion problem. In particular, the structured information can be easily parsed for downstream applications, including the integration of additional classical algorithms, or prompting the user to provide the yet missing information, if needed. The structured information may comprise a set of structured information items. These could be represented, for example, by codes from commonly used coding systems (ICD-10, LOINC, CPT, ...) and/or custom, task-specific representations. In particular, the structured information may comprise codes, e.g., ICD-10 or CPT codes, despite the codes not being present in the natural language data.

The medical imaging decision support data may be calculated, for example, by applying a function for calculating the medical imaging decision support data onto the structured information. The function for calculating the medical imaging decision support data may be trained, in particular machine-learned. For example, the large language model may comprise the function for calculating the medical imaging decision support data. As an alternative, the function for calculating the medical imaging decision support data may be rule-based.

A specific advantage of the proposed method results from its strong comprehensibility. As information is presented in a highly structured format, it is easily possible to spot inconsistent information through a human, and thus to reduce novel sources of error as they might result from ontology-based approaches. Further, the reconfigurability of the large language model allows for a simple adaption in case of updated report templates, novel tasks and diseases, and finally the update of previously created structured reports, as reconfigurations correspond to changes in the query, but not changes in the architecture of the large language model.

The large language model may comprise a transformer network. A transformer network is a neural network architecture generally comprising an encoder, a decoder or both an encoder and decoder. In some instances, the encoders and/or decoders are composed of several corresponding encoding layers and decoding layers, respectively. Within each encoding and decoding layer is an attention mechanism. The attention mechanism, sometimes called self-attention, relates data items (such as words or pixels) within a series of data items to other data items within the series. The self-attention mechanism for instance allows the model to examine a word within a sentence and determine the relative importance another word within that sentence has to the word being examined.

The encoder, in particular, may be configured to transform the input (a medical image or text) into a numerical representation. The numerical representation may comprise a vector per input token (e.g., per word). The encoder may be configured to implement an attention mechanism so that each vector of a token is affected by the other tokens in the input. In particular, the encoder may be configured such that the representations resolve the desired output of the transformer network. The decoder, in particular, may be configured to transform an input into a sequence of output tokens. In particular, the decoder may be configured to implement a masked self-attention mechanism so that each vector of a token is affected only by the other tokens to one side of a sequence. Further, the decoder may be auto-regressive meaning in that intermediate results (such as a previously predicted sequence of tokens) are fed back. According to some examples, the output of the encoder is input into the decoder. Further, the transformer network may comprise a classification module or unit configured to map the output of the encoder or decoder to a set of learned outputs such as the text summary.

Training of a transformer model according to some examples may happen in two stages, a pretraining and a fine-tuning stage. In the pretraining stage, a transformer model may be trained on a large corpus of data to learn the underlying semantics of the problem. Such pre-trained transformer models are available for different languages. For certain applications described herein, the fine-tuning may comprise further training the transformer network with medical texts with expert annotated meanings and/or medical ontologies such as RADLEX and/or SNOMED. With the latter, in particular, the transformer model according to some examples may learn typical relations and synonyms of medical expressions.

The large language model may comprise, for example, a transformer architecture. Transformer networks may use the previously inserted text to find a logical continuation by querying the already provided information, extracting key information, and assigning a value to each. Conducting this process layer by layer, the large language model predicts one token (i.e., word or word part) at a time, with each token being based on the provided information, as well as the world knowledge that is implicitly represented in the network weights.

A query may be received, the query comprising the natural language data and further query data, wherein the structured information is generated by applying the large language model onto the natural language data and the further query data. The query may comprise, for example, a user query. The user query may be a free text query the user may input into a user interface, e.g., by typing the user query into an appropriate input filed in the user interface or by voice command.

The further query data may comprise template data, the template data being indicative of the structured format. In particular, the large language model may be provided with a dynamically configurable template and queried to transform the natural language data, thereby filling the relevant information into the provided template.

Reference data may be received, the reference data being indicative of rules and/or examples related to the generating the structured information and/or related to the calculating the medical imaging decision support data, wherein the large language model is tuned based on the reference data, in particular self-tuned and/or fine-tuned based on the reference data.

The large language model may be configured to consider the reference data as an additional source of information, in particular in form of general medical guidelines, site-specific rules and/or preferences, and/or the patient history. The examples may be related to different patients and/or examinations. In particular, the reference data may comprise a corresponding preferred medical imaging decision for different sets of exemplary patient-specific clinical information. In particular, the further query data may comprise the reference data. In particular, the reference data may be automatically injected as part of the query. Based on the reference data, the large language model may identify patterns and/or reasons why certain recommendations for a new patient and/or exam are desirable, thus allowing, for example, for an on-the-fly learning.

Explanation data may be calculated by the large language model, the explanation data being indicative of an explanation and/or a reason for the generating the structured information and/or for the calculating the medical imaging decision support data, wherein the explanation data are provided.

The large language model may comprise a function for explaining, in particular interactive explaining, and/or reasoning, in particular interactive reasoning, in particular about the structured information and/or about the medical imaging decision support data. As a key ability, typical large language models are inherently able to explain their decisions with respect to the context that they are applied in. The explanation data may be further indicative of an explanation and/or a reason for a tuning of the large language model based on the reference data. The explanation data may be automatically documented and/or made directly accessible to a user, e.g., as a hover overlay, a decision log and/or an info button and/or through interactive querying, which allows for cross-checking the automated inference, for example, in form of a natural language dialogue.

For example, the explanation data may comprise a citation of content from the natural language data, in particular from an unstructured report, as a verifiable source for the generated structured information and/or for the calculated medical imaging decision support data. For each citation of content from the natural language data, the explanation data may comprise a citation-specific relevance information and/or citation-specific context information. The context information may comprise, for example, references to guidelines, rules, preferences, patient history and/or examples (if any), in particular together with an outline of a relevance of each reference for the given case.

Examination request data may be received, the examination request data being indicative of a type of a medical imaging examination, the medical imaging decision support data being indicative of an appropriateness of the type of the medical imaging examination in view of the patient-specific clinical information.

When a scan is ordered by the referring physician, it is mandatory to check that the requested type of scan is appropriate for the given indication. Given the text-based referrer request and a description of the appropriate scan for the indication, the large language model can perform this check with only minimal interaction, which is particularly relevant if the user disagrees with the assessment. Moreover, the large language model can be queried regarding the reasoning for this decision, in order to quickly determine if there was a human oversight, or if this may be an erroneous conclusion by the large language model, e.g., due to missing information.

A medical imaging examination workflow step, option and/or parameter may be suggested, selected and/or triggered, in particular automatically suggested, selected and/or triggered, based on the medical imaging decision support data.

The medical imaging decision support data may be indicative of a value and/or a value change of a scan parameter of a scan protocol for a medical imaging examination of a patient by a medical imaging device. The method for providing medical imaging decision support data may further comprise: - setting the scan parameter and/or conducting a medical imaging examination based on the scan parameter.

The medical imaging decision support data may be indicative of a value and/or a value change of a reconstruction parameter of a reconstruction algorithm for reconstructing a medical image based on medical imaging data. The method for providing medical imaging decision support data may further comprise: - setting the reconstruction parameter and/or reconstructing the medical image based on the medical imaging data.

The medical imaging decision support data may be indicative of a value and/or a value change of an image processing parameter of an image processing algorithm for processing a medical image. The method for providing medical imaging decision support data may further comprise: - setting the image processing parameter and/or processing the medical image. The image processing algorithm may be configured, for example, for post-processing and/or analysis of the medical image. The post-processing of the medical image may comprise, for example, calculating a representation of an anatomical structure based on the medical image.

The structured information can be used, for example, to enhance and/or automate a scan workflow and/or a post-processing workflow. For this purpose, information from the structured report and the unstructured report as well as the referral request may be automatically structured with respect to the clinical questions relevant to the medical imaging exam to be performed, while including all specific aspects surrounding this concern. Exemplarily, based on the given textual information, the large language model can be used to quickly answer relevant questions such as "May the patient receive contrast agent?", "Is a high-resolution reconstruction recommended for the given indication", "In which organs should an automated search for metastases be performed?", and similar.

The structured information, in particular regarding patient demographics, previous findings or comorbidities, may be used, for example, for selecting and/or triggering the appropriate workflows steps, for choosing appropriate parameters for scanning, reconstruction and post-processing, and as prior information for any machine-learning based image processing algorithm run on the resulting image data set.

When the large language model is trained to take this additional input information into account, its accuracy is expected to increase and the results can also be automatically checked for consistency with the available information, e.g., to decrease the chance of false positive findings.

The method may be implemented in form of a Tech Co-pilot supporting technologists in their daily work. The method allows to automatically determine relevant protocol refinement steps given prior reports and a clinical indication, i.e., the reason for exam. These data may be acquired, for example, by utilizing open standards, such as the DICOM Modality Worklist and the FHIR standard. Using this data, however, the suggested Tech Co-pilot would allow for automatic protocol refinement, comprising scanner options such as image coverage, reconstruction and reformatting, multiple energy, spectral and multi-phase acquisition, kernel choice, dose and exposure control (Care keV), bolus application, timing and tracking, inspiratory or expiratory scanning, (quantum) iterative reconstruction and ADMIRE parameterization, metal & motion artifact reduction, and subsequent CAD applications.

For patients with previous written medical records and given the screening guidelines, the method may be used to identify patients which are eligible for and could benefit from a lung screening most. If the decision to perform a screening exam has been made by a clinician, this information is present in the referral text as a "reason for exam". It can thus be interpreted as such by the large language model.

Based on this information, the appropriate scan protocol may be automatically determined based on the method for providing medical imaging decision support data, i.e., a specific ultra-low dose protocol is used for screening, in order to avoid undue radiation exposure in likely healthy patients - and an appropriate Lung CAD algorithm is automatically triggered that detects potential lung nodules in the acquired image. In case of a scanner that allows for the acquisition of spectral information such as photon counting scanners, a quantitative evaluation of the composition of any detected lesions for further characterization may also be triggered automatically. Finally, the large language model is used to summarize the results and findings, and/or details them in a structured report.

Cancer patients regularly receive follow-up scans for staging to determine the progress of the disease as well as the effectiveness of the treatment. Thus, typically several prior diagnostic reports are available that detail, amongst others, the primary cancer type, the identified target lesions, and finally all known metastatic loci. This information may be forwarded to the large language model. The method for providing medical imaging decision support data may further comprise at least one of
- summarizing the disease progression up to the current point in time, making it readily available as a reference for the current exam,
- identifying the most appropriate baseline scan and relevant target lesions therein, by comparing with the current exam using lesion detection and segmentation algorithms (that may themselves be provided with the information about known lesion positions to increase the confidence of their results), and automatically supporting the calculation of quantitative measures such as RECIST,
- triggering the generation of appropriate visualizations, for example, in case of pancreatic cancer patients, high-resolution stacks in standard orientations, including iodine overlay maps as well as unfolded images of the organ and its surrounding vessels,
- determining organs that are likely to be affected by metastatic lesions and automatically trigger appropriate lesion detection algorithms,
- summarizing the current findings in the structured report data and contrast them with the previous reports, and/or
- summarizing the clinical guidelines for staging of the corresponding type of cancer and answer specific questions about how the staging should be performed.

The invention further relates to a data processing system for providing medical imaging decision support data, the data processing system comprising an interface unit and a computing unit,
- wherein the interface unit is configured to receive natural language data, the natural language data comprising patient-specific clinical information,
- wherein the computing unit is configured to generate structured information by applying a large language model onto the natural language data, the structured information comprising the patient-specific clinical information in a structured format,
- wherein the computing unit is further configured to calculate the medical imaging decision support data based on the structured information, and
- wherein the interface unit is further configured to provide the medical imaging decision support data.

The data processing system may be configured for carrying out the method according to one of the aspects of the invention.

The data processing system can comprise, for example, at least one of a cloud-computing system, a distributed computing system, a computer network, a computer, a tablet computer, a smartphone or the like. The data processing system can comprise hardware and/or software. The hardware can be, for example, a processor system, a memory system and combinations thereof. The hardware can be configurable by the software and/or be operable by the software. Calculations for performing an action of a method may be carried out in the processor.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the invention. The hardware can be configurable by the software and/or be operable by the software. Generally, all units, sub-units, or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the medial image data sets. The interface unit may be further adapted to interface with one or more users of the system, e.g., by displaying the result of the processing by the computing unit to the user (e.g., in a graphical user interface) or by allowing the user to adjust parameters for data processing or visualization. In other words, the interface unit may comprise a user interface.

Data, in particular the natural language data, the query, the further query data, the reference data and the examination request data, can be received, in particular received through the interface unit, for example, by receiving a signal that carries the data and/or by reading the data from a computer memory and/or by a manual user input, for example, through a graphical user interface. Data, in particular the medical imaging decision support data and/or the explanation data, can be provided, in particular provided through the interface unit, for example, by transmitting a signal that carries the data and/or by writing the data into a computer memory and/or by displaying the data on a display.

Any of the algorithms, functions and/or models mentioned herein can be based on one or more of the following architectures: deep convolutional neural network, deep belief network, random forest, deep residual learning, deep reinforcement learning, recurrent neural network, Siamese network, generative adversarial network or auto-encoder.

The invention further relates to a medical imaging device comprising the data processing system according to one of the aspects of the invention. The medical imaging device may be configured for carrying out the medical imaging examination of the patient based on the scan protocol for the medical imaging examination. The medical imaging device may be, for example, a computed tomography device, a magnetic-resonance imaging device, an ultrasound imaging device, a PET imaging device, a SPECT imaging device, an X-ray imaging device, a cone beam CT device and/or a combination thereof.

The invention further relates to a computer program product, comprising instructions which, when the instructions are executed by a computer, cause the computer to carry out the method according to one of the aspects of the invention. The invention further relates to a computer-readable storage medium, comprising instructions which, when the instructions are executed by a computer, cause the computer to carry out the method according to one of the aspects of the invention. The realization of the invention by a computer program product and/or a computer-readable storage medium has the advantage that already existing computers can be easily adapted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example, a documentation or a software key for using the computer program. The computer program product may further comprise development material, a runtime system and/or databases or libraries. The computer program product may be distributed among several computer instances. A computer-readable storage medium can be embodied as non-permanent main memory (e.g. random-access memory) or as permanent mass storage (e.g. hard disk, USB stick, SD card, solid state disk).

Utilizing a large language model capable of language understanding, rather than only parsing, poses a significant advantage in comparison to symbolic-ontological methods, that typically struggle with negations, inversions, abbreviations, typos and extra information, either being parsed "hardcoded", and thus very limited, or without being able to handle this information at all. In contrast, language understanding, information extraction and content summarization are key capabilities of typical large language models, allowing to avoid these issues. Specifically, while negations and inversions are an integral part of natural language and thus abundantly represented, world knowledge as well as explicitly given query information can be used to resolve abbreviations and typos. Extra information can be explicitly queried and summarized, such as in form of easily comprehensible bullet points, which themselves may refer to the original report.

As a result, the use of large language models might revolutionize the way in which structured information is created from unstructured natural language data, being a major step toward digital transformation in clinics, improving throughput, accelerating treatment, serving as a double-check for avoiding treatment errors, and finally reducing the workload for the involved parties. With a specific focus on the scan and post-processing workflow automation, such as addressed by the Tech Co-pilot, the advantages are similarly manifold, with the main aspects including time savings through smart scanners, automatically pre-selecting correct workflows and steps, and triggering only relevant and specifically tailored algorithms in order to create relevant (AI-based) image analysis results, standardization, and increased consistency of exams. In summary, this allows for highly reproducible, guidance-based workflow recommendations, and results in a strong potential for error reduction by serving as an automated "second pair of eyes" that is automatically parsing the available text-based sources for any relevant information.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature.

Reference is made to the fact that the described methods and the described systems are merely preferred example embodiments of the invention, and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a flow chart for a computer-implemented method for providing medical imaging decision support data.
Fig. 2 shows the data processing system.
Fig. 3 shows a data flow diagram through an information system.
Fig. 4 shows a medical imaging workflow environment.
Fig. 5 shows a table of a structured report.

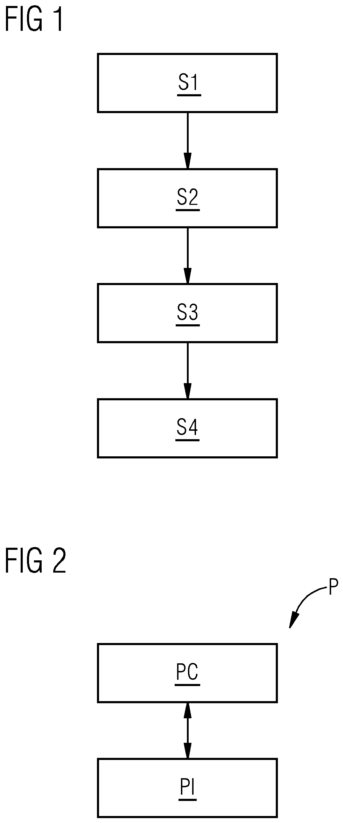

Fig. 1 shows a flow chart for a computer-implemented method for providing medical imaging decision support data, the method comprising:
- receiving S1 natural language data U, the natural language data U comprising patient-specific clinical information,
- generating S2 structured information N by applying a large language model onto the natural language data U, the structured information N comprising the patient-specific clinical information in a structured format,
- calculating S3 the medical imaging decision support data based on the structured information N, and
- providing S4 the medical imaging decision support data.

Fig. 2 shows the data processing system P for providing medical imaging decision support data, the data processing system P comprising an interface unit PI and a computing unit PC,
- wherein the interface unit PI is configured to receive S1 natural language data U, the natural language data U comprising patient-specific clinical information,
- wherein the computing unit PC is configured to generate S2 structured information N by applying a large language model onto the natural language data U, the structured information N comprising the patient-specific clinical information in a structured format,
- wherein the computing unit PC is further configured to calculate S3 the medical imaging decision support data based on the structured information N, and
- wherein the interface unit PI is further configured to provide S4 the medical imaging decision support data.

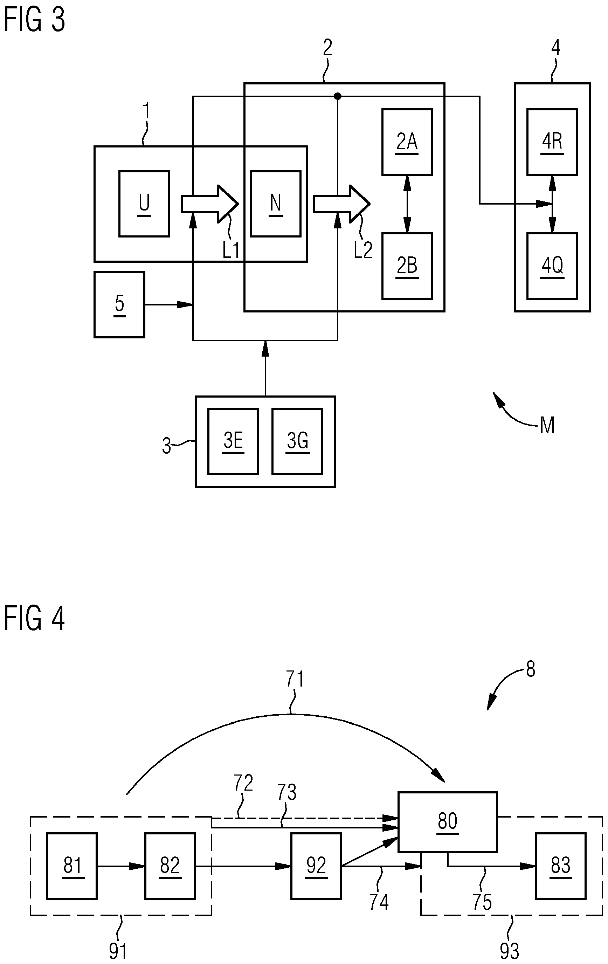

Fig. 3 shows a data flow diagram through the information system M, the large language model being implemented in the information system M. The information system M may be, for example, a computer information system M. Therefore, the information system M is able to gracefully handle almost all the idiosyncrasies of natural language as used in the day-today clinical routine.

The information system M comprises the component 1 for transforming unstructured information to structured information, the component 2 for medical imaging workflow automation and enhancement, the component 3 for on-the-fly learning and the component 4 for explainable processing. Each of this components utilizes Large Language Model (LLM)-based processing for result inference. The large language model of the information system M comprises the function L1 for generating S2 the structured information N based on the natural language data U and the function L2 for calculating S3 the medical imaging decision support data based on the structured information N. The large language model of the information system M further comprises the function 4R for explaining and/or reasoning. The component 4 comprises the user interface 4Q for interactive querying.

The component 2 comprises the module 2A for suggesting, selecting and/or triggering a medical imaging examination workflow step, option and/or parameter related to acquisition, in particular related to the scan protocol, and/or related to reconstruction, in particular related to the reconstruction algorithm. The component 2 further comprises the module 2B for suggesting, selecting and/or triggering a medical imaging examination workflow step, option and/or parameter related to image processing, in particular related to the image processing algorithm. For example, the module 2B may comprise an interface for injecting the structured information as a prior information input to the image processing algorithm.

Reference data are received, the reference data being indicative of rules 3G and/or examples 3E related to the generating S2 the structured information N and/or related to the calculating S3 the medical imaging decision support data, wherein the large language model is tuned based on the reference data. The component 3 is configured for tuning the large language model based on reference data, the reference data being indicative of the rules 3G, in particular in form of general medical guidelines, site-specific rules and/or preferences, and/or being indicative of the examples 3E. The component 3 may be further configured for tuning the large language model based on the patient history data 5. Therefore, the information system M is able to learn from and reason with context information and prior examples on-the-fly as described above.

Fig. 4 shows the medical imaging workflow environment 8. The information system M may be used as a Tech Co-pilot 80 for the technologist 83 in the medical imaging workflow environment 8. Given a referral by the physician 81, the referral comprising an imaging request to resolve a clinical question (indication), the radiologist 82 determines an appropriate base protocol. As a communication platform 91 for the physician 81 and the radiologist 82, an electronic medical record (EMR) and/or a radiology information system (RIS) may be used.

The appropriate base protocol is documented, e.g., in the DICOM Modality Worklist 92 and forwarded to the medical imaging device 93 in form of a requested procedure 74 with technical terms only, for example "CT Thorax w/o contrast". In addition, background information regarding the protocol choice 71, for example "Long nodule follow up CT", may be directly forwarded from the physician 81 and/or the radiologist 82 to the technologist 83. The reason 73 for the exam, for example "Pulmonary nodules follow-up" is provided to the Tech Co-pilot 80. Receiving additional information such as prior diagnostic reports 72 through communication interfaces such as FHIR, the Tech Co-pilot 80 infers and suggests relevant protocol refinements 75 for the technologist 83, significantly reducing workload for adjusting scan, reconstruction and/or post-processing protocols. The medical imaging device 93 comprises the data processing system P.

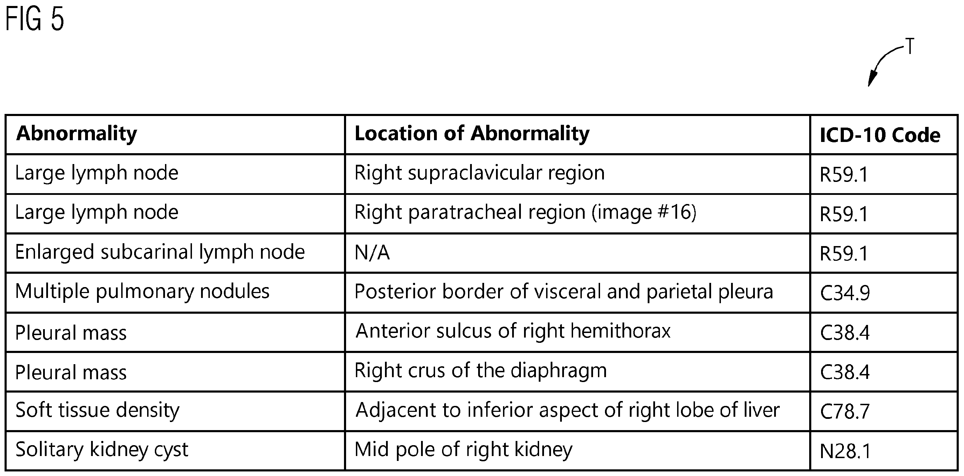

Fig. 5 shows the table T of a structured report. For example, the transformer-based large language model of ChatGPT is capable of generating a structured report based on the following query, the query comprising unstructured natural language data and a report template.

"Extract the findings from the following radiological report and summarize the findings in the following template:
Name:
Age:
Gender:
Medical history:
   <bullet points>
Findings:
   <a table with three columns - abnormality, location of abnormality, corresponding ICD-10 code>
Summary:
   <bullet points>

CLINICAL HISTORY: John Doe, a 68-year-old white male with recently diagnosed adenocarcinoma by sputum cytology. An abnormal chest radiograph shows right middle lobe infiltrate and collapse. Patient needs staging CT of chest with contrast. Right sided supraclavicular and lower anterior cervical adenopathy noted on physical exam.

TECHNIQUE: Multiple transaxial images utilized in 10 mm sections were obtained through the chest. Intravenous contrast was administered.

FINDINGS: There is a large 3 x 4 cm lymph node seen in the right supraclavicular region. There is a large right paratracheal lymph node best appreciated on image #16 which measures 3 x 2 cm. A subcarinal lymph node is enlarged also. It measures 6 x 2 cm. Multiple pulmonary nodules are seen along the posterior border of the visceral as well as parietal pleura. There is a pleural mass seen within the anterior sulcus of the right hemithorax as well as the right crus of the diaphragm. There is also a soft tissue density best appreciated on image #36 adjacent to the inferior aspect of the right lobe of the liver which most likely also represents metastatic deposit. The liver parenchyma is normal without evidence of any dominant masses. The right kidney demonstrates a solitary cyst in the mid pole of the right kidney.

### IMPRESSION:

1. Greater than twenty pulmonary nodules demonstrated on the right side to include pulmonary nodules within the parietal as well as various visceral pleura with adjacent consolidation most likely representing pulmonary neoplasm.
2. Extensive mediastinal adenopathy as described above.
3. No lesion seen within the left lung at this time.
4. Supraclavicular adenopathy."

The following is the reply to the above query.
"Name: John Doe
Age: 68
Gender: Male
Medical history:
   - Recently diagnosed adenocarcinoma by sputum cytology
Findings:
   Table T shown in FIG 5.

Summary:
- Presence of greater than twenty pulmonary nodules on the right side, including nodules within the parietal and visceral pleura, with adjacent consolidation, likely indicative of pulmonary neoplasm.
- Extensive mediastinal adenopathy with enlarged lymph nodes in the right supraclavicular region, right paratracheal region, and subcarinal region.
- No lesion observed within the left lung at this time.
- Supraclavicular adenopathy."

## Claims

1. A computer-implemented method for providing medical imaging decision support data, the method comprising:
- receiving (S1) natural language data (U), the natural language data (U) comprising patient-specific clinical information,
- generating (S2) structured information (N) by applying a large language model onto the natural language data (U), the structured information (N) comprising the patient-specific clinical information in a structured format,
- calculating (S3) the medical imaging decision support data based on the structured information (N), and
- providing (S4) the medical imaging decision support data.

2. The method according to claim 1,
- wherein the large language model comprises a transformer network.

3. The method according to claim 1 or 2,
- wherein a query is received, the query comprising the natural language data (U) and further query data,
- wherein the structured information (N) is generated by applying the large language model onto the natural language data (U) and the further query data.

4. The method according to claim 3,
- the further query data comprising template data, the template data being indicative of the structured format.

5. The method according to one of the claims 1 to 4,
- wherein reference data are received, the reference data being indicative of rules (3G) and/or examples (3E) related to the generating (S2) the structured information (N) and/or related to the calculating (S3) the medical imaging decision support data,
- wherein the large language model is tuned based on the reference data.

6. The method according to one of the claims 1 to 5,
- wherein explanation data are calculated by the large language model, the explanation data being indicative of an explanation and/or a reason for the generating (S2) the structured information (N) and/or for the calculating (S3) the medical imaging decision support data,
- wherein the explanation data are provided.

7. The method according to one of the claims 1 to 6,
- wherein examination request data are received, the examination request data being indicative of a type of a medical imaging examination,
- the medical imaging decision support data being indicative of an appropriateness of the type of the medical imaging examination in view of the patient-specific clinical information.

8. The method according to one of the claims 1 to 7,
- wherein a medical imaging examination workflow step, option and/or parameter is suggested, selected and/or triggered based on the medical imaging decision support data.

9. The method according to one of the claims 1 to 8,
- the medical imaging decision support data being indicative of a value and/or a value change of a scan parameter of a scan protocol for a medical imaging examination of a patient by a medical imaging device.

10. The method according to one of the claims 1 to 9,
- the medical imaging decision support data being indicative of a value and/or a value change of a reconstruction parameter of a reconstruction algorithm for reconstructing a medical image based on medical imaging data.

11. The method according to one of the claims 1 to 10,
- the medical imaging decision support data being indicative of a value and/or a value change of an image processing parameter of an image processing algorithm for processing a medical image.

12. A data processing system (P) for providing medical imaging decision support data, the data processing system (P) comprising an interface unit (PI) and a computing unit (PC),
- wherein the interface unit (PI) is configured to receive (51) natural language data (U), the natural language data (U) comprising patient-specific clinical information,
- wherein the computing unit (PC) is configured to generate (S2) structured information (N) by applying a large language model onto the natural language data (U), the structured information (N) comprising the patient-specific clinical information in a structured format,
- wherein the computing unit (PC) is further configured to calculate (S3) the medical imaging decision support data based on the structured information (N), and
- wherein the interface unit (PI) is further configured to provide (S4) the medical imaging decision support data.

13. A medical imaging device (93) comprising the data processing system (P) of claim 12.

14. A computer program product, comprising instructions which, when the instructions are executed by a computer, cause the computer to carry out the method of one of the claims 1 to 11.

15. A computer-readable storage medium, comprising instructions which, when the instructions are executed by a computer, cause the computer to carry out the method of one of the claims 1 to 11.
